# EUROPEAN PATENT SPECIFICATION

(11) **EP 1 755 506 B1**
(45) Date of publication and mention of the grant of the patent: **14.05.2008**
(21) Application number: 05736148.7
(22) Date of filing: 29.04.2005
(51) Int. Cl.: A61F 5/44

(54) **An ostomy appliance**
Stomavorrichtung
Accessoire de stome

(30) Priority: 03.05.2004 DK 200400699
(43) Date of publication of application: 28.02.2007
(73) Proprietor: COLOPLAST A/S, 3050 Humlebaek (DK)
(72) Inventor: WORSOEE, Bjarne, DK-3080 Tikoeb (DK)
(86) International application number: PCT/DK2005/000292
(87) International publication number: WO 2005/105006

(56) References cited:
- EP-A- 0 209 347
- EP-A- 0 263 975
- GB-A- 2 177 301
- US-A- 4 938 750

## Description

### Field of the invention

The present invention relates to an ostomy appliance comprising an openable passage. In particular the present invention relates to an ostomy appliance comprising a wall with a passage zone adapted to change from a closed state to an open state, when exposed to a waste material.

### Background of the invention

In connection with ostomy appliances a problem is that the discharge of flatus, measured in volume, may exceed the discharge of solid and liquid faecal matter by many hundred percent and therefore there is usually the need for continuous or frequent venting of the intestine or the collecting bag. Normally the outflowing flatus is deodorised with a suitable filter.

However one problem in connection with filters is that the filter may be blocked due to the waste material i.e. the solid and liquid faecal matter. Efforts have been made to overcome the problem and systems wherein the filter may be changed manually by the user have been designed.

It is known in the art to provide ostomy appliances with passages initially being closed and which manually may be changed from the closed state to an open state. One example may be found in US 4,938,750 which describes an ostomy collection pouch comprising a pouch wall which has a plurality of vent passages each containing a filter. Each filter can be exposed in turn simply by moving an adhesive cover from one location to the next. The adhesive cover is provided on the outside such that it is possible for the user to move the adhesive cover. Thus as the surface of the filter facing the interior of the pouch is not covered prior to use, a filter may be blocked from the inside before the filter has even been used.

Furthermore it is known from EP 0 209 347 that a filter may be removed manually. A filter is attached to the outer surface of the front wall by means of a series or stack of sequentially removable annuli. Each of these has adhesive on it. When the wearer believes that the filter may need replacement he removes the tab. In this way the filter is removed and thereafter a new filter may be pressed on to the exposed fresh adhesive. Accordingly in the period between removal of the old filter and application of the new filter, flatus gasses may escape the ostomy appliance.

It may be seen as an object of the present invention to provide a system which protects a filter from contamination prior to its use.

Additionally it may be seen as an object of the present invention to utilize the wet and bacteria-containing environment inside the ostomy appliance to open a closed wall e.g. provide passage through the filter

### Description of the invention

The present invention relates to an ostomy appliance comprising a collecting bag having a front wall and a rear wall, the rear wall having an opening into the bag through which waste material may enter the collecting bag, and wherein at least one of the walls comprises at least one passage zone adapted to change from a closed state to an open state, when exposed to the waste material.

One advantage of the invention is that as the filter is provided inside the ostomy appliance and not on the outer surface, the filter is not removed by accident whereby flatus gasses could escape the appliance.

Another advantage of the present invention is that the ostomy appliance is adapted to provide a free passage through a filter without the user's intervention.

In the closed state the passage zone defines a passage wall. In the open state the passage wall is decomposed or dissolved whereby the passage zone is open. The passage wall may in the closed state prevent gas and/or liquid from passing through the wall. The passage zone may also prevent light from passing through the passage wall whereby an indicator provided behind the passage wall may not be seen. In the open state the passage zone may be adapted to allow light and/or gas and/or liquid to pass through a hole or aperture which has been provided as the passage wall is decomposed or dissolved. In one embodiment the passage zone is adapted to allow light to pass through the passage wall when the passage zone is in the open state while gasses and/or liquid cannot pass. In other embodiments only liquid cannot pass through the passage zone when the passage zone is in the open state. In the two latter embodiments the passage wall may not be completely dissolved but has only changed properties such that light or gasses may pass through the passage wall.

In some embodiments the entire passage wall is dissolvable. In other embodiments the passage zone in the open state may be provided as a plurality of holes in a predetermined area of the passage wall. Such a plurality of holes may be provided in a predetermined pattern or in a random pattern.

The passage zone may be provided in the same kind of material as the rest of the front and rear walls or in a different material. If the passage zone is provided in the same kind of material as the rest of the wall the passage zone may be processed in a certain way at some point of the manufacturing process. Such processing could be coating, heat treatment, chemical treatment etc.

Alternatively, the front or rear wall are made of a dissolvable or decomposable material and covered with a sealing material except from the area of the passage zone. Thus, waste material may get in contact with the dissolvable or decomposable material in the area of the passage zone but is prevented from dissolving or decomposing the rest of the front and rear wall.

In yet another embodiment the entire front and rear wall including the area of the passage zone, is made of the same kind of material and the passage zone (which is provided in one of said walls) is coated with a material e.g. containing an enzyme which is adapted to initiate the dissolving of the passage zone. In the latter embodiment the passage zone could also be coated with a chemical compound which when brought into contact with liquid turns into an acid. Such a chemical compound could be an acid an-hydride. In a further embodiment the passage zone is adapted to change from the closed to the open state when exposed to the heat of the waste material.

The collecting bag may be made as collecting bags known per se, e.g. the bag may comprise a front wall and a rear wall each made of sheet material attached to each other. The walls may be glued, welded etc. together.

The waste material may contain any combination of solids, semi-solids, liquids and gas. If the waste material contains gas, the passage zone may be used to open a passage into a deodorizing filter.

In one embodiment at least a part the passage zone is adapted to decompose and/or dissolve when exposed to the waste material. One or more parts of the passage zone may be adapted to dissolve or decompose. In some embodiments the material of the passage zone changes from solid form to liquid form whereby the material (in liquid form) is mixed with the waste material. In other embodiments the material of the passage zone is decomposed such that it changes form e.g. due to a chemical reaction or decomposition by bacteria.

The passage zone may be decomposable by bacteria and/or by enzymes. The bacteria may be provided in the waste material. Alternatively the bacteria may be applied to the surface of the passage zone and/or may be provided in the material of the passage zone. In the latter embodiments the bacteria stays inactive until influenced by the heat and/or the humidity of the waste material and thus bacteria in or on the passage zone will not start to decompose the passage zone prior to the use of the appliance. If more passage zones are provided on the surface or material may be provided with different types of bacteria or enzymes such that two passage zones are dissolved or decomposed at different speed.

Dissolvable materials may comprise any combination of polyvinyl alcohol (PVOH), polyacrylic acid or derivatives, or hydrocolloides such as carboxymethylecellulose og Hydroxyethylecellulose.

Decomposable materials may comprise starch based polymers (usually composition of starch, polylactic acid and glycerol). Starch can be corn, potatoe ect. or polyesterbased materials (polylactic acid).

The passage zone may be a combination of the dissolvable and decomposable materials. In one example two passage zones are provided, a first comprising a dissolvable material, and a second comprising two layers an outer dissolvable layer and an inner decomposable layer. The dissolvable material of the two passage zones may be dissolved after same time of exposure to the waste material. However as the second passage zone also comprises a decomposable material it will change from the closed state to open state later than the first passage.

In one embodiment the surface of each passage zone is - in the closed stage - stretched. Thus one hole in the passage zone may cause the rest of the passage zone to rupture and thus open the passage zone faster than if the entire passage zone had to dissolve or decompose. In one embodiment a ring shaped part of the passage zone is provided in a thinner material than the rest of the passage zone. Thus the thinner area will be dissolved or decomposed faster than the rest of the passage zone whereby the entire passage zone opens in two stages.

In yet another embodiment one or more passage zones are made in a material with memory. As the material changes form when exposed to water and/or heat, the passage zone may change from the closed state to the open state when exposed to water or heat.

The surface of at least one passage zone may comprise a penetrable layer adapted to allow penetration of the liquid and a creasable layer adhered to the penetrable layer by means of a dissolvable adhesive. Thus when waste material enters the collecting bag the waste material will pass through the penetrable layer and dissolve the dissolvable adhesive. After a period of time the creasable layer will not be adhered to the penetrable layer and thus the creasable layer will crease whereby the passage zone will open.

In another embodiment the passage zone comprises two layers of a moister sensitive material and a moister penetrable material. When the waste material enters the collecting bag it will start to pass through the penetrable material and dissolve the moister sensitive material. When the moister sensitive material has been dissolved the passage zone will be open and flatus gasses may pass from the interior of the collecting bag through the passage zone and thereafter a filter and escape the appliance through a exit hole or membrane provided on the outer surface of the appliance. If a plurality of passage zones is provided the moister penetrable material of two passage zones may be different in order to open the passage zones at different speeds. Alternatively the dissolvable material may provided in different thicknesses in order to have different opening times of the passage zones.

In a further embodiment the collecting bag comprises a first compartment and a second compartment separated by a passage zone. In the latter embodiment the first compartment may comprise the opening through which the waste material enters the ostomy appliance. When the waste material starts to enter the appliance the passage zone separating the first compartment and the second compartment will start to dissolve or decompose. After a period of time the first compartment will not be big enough to contain the waste material and if not expanded it will start to balloon. However at this time the passage zone will be decomposed or dissolved and thus the waste material may enter the second compartment. Accordingly it is possible to provide an ostomy appliance which initially is small and discrete in size and which automatically will expand after a predetermined time of use.

The ostomy appliance may comprise a filter through which gasses may pass. The filter may be provided such that the passage zones are provided on an internal surface of the collecting bag e.g. such that a part of the rear wall and/or front wall cover the filter. The filter may be provided in a separate compartment.
A deodorizing filter may be provided in the separate compartment. Furthermore a pre-filter may be provided in the separate compartment. The pre-filter can be adapted to prevent waste material from contaminating the deodorizing filter. A membrane may be provided which prevents waste material from escaping the ostomy appliance. The order of the elements in the separate seen from the inside of the collecting bag may be pre-filter, deodorizing filter and membrane. However the elements may be placed in any sequence.

In one embodiment the ostomy appliance is designed such that the passage zone in the open state allows gasses to pass through the filter. In the initial state the area of the wall covering the filter may be provided with an initial hole and a passage zone that is closed. The gasses may in the beginning pass through the initial hole and be filtered in the filter. After a period of time a part of the filter in the area of the initial hole may be blocked by the waste material and the consequence is that the gasses cannot escape the collecting bag. After a further period of time, the passage zone may open and thus provide an alternative route for the gasses. Different filters may be provided such that the filter used in connection with the initial hole is different than the filter used in connection with the open passage zone.

The ostomy appliance may be provided with one or more passage zones. In one embodiment the ostomy appliance comprises two passage zones and optionally an initial hole. The two passage zones may be denoted the first passage zone and the second passage zone. The first and second passage zone may be adapted to open at different times.

The first passage zone may be adapted to change from the closed state to the open state after being exposed to the waste material for a predetermined first period of time. The second passage zone may be adapted to change from the closed state to the open state after being exposed to the waste material for a predetermined second period of time. The first period of time may be shorter than the second period of time.

In the following the latter embodiment of the invention is described in further detail. Initially the collecting bag is provided with the initial hole and the two closed passage zones - the first and second passage zone. The waste material enters the collecting bag and gasses from the waste material escape the collecting bag through the initial hole and are filtered in the filter of the ostomy appliance. After a period of time a part of the filter in the area of the initial hole is blocked by the solid or semi-solid material of the waste material. The result is that gasses may not escape the collecting bag through the initial hole and the collecting bag will start to balloon i.e. pressure may start to build up inside the collecting bag whereby it inflates.

However the first passage zone will open after the predetermined first period of time as the wall of the first passage zone will dissolve or decompose as result of being exposed to (i.e. being in contact) the waste material. When the first passage zone is open the gasses of the waste material may escape the collecting bag through the first passage zone, preventing ballooning of the collecting bag. However the area of the filter adjacent to the first passage zone will slowly start to be blocked by the waste material, whereby the gasses may not escape the collecting bag through the first passage zone. At this stage the second passage zone may be dissolved and thus allow gasses to pass through the second passage zone.

In the above description of the first passage zone is not opened before the initial hole is blocked and the second passage zone in not opened before the first passage zone is blocked. In some embodiments the blockage and opening will overlap each other and thus the initial hole will slowly be increasingly blocked while a passage zone is slowly opening. In some embodiments two passages will be open at the same time. The exact timing of the opening and closing of the initial hole and the passage zones will depend on the type and flow rate of waste material, accordingly the exact timing is difficult to predict/design.

In order to provide passage zones which opens at different times, the thickness of the wall of the first passage in the closed state may be smaller than the thickness of the wall of the second passage in the closed state. The difference in thickness may be provided by using the same kind of material from two different kinds of sheet having different thicknesses.

Another way of providing the different thicknesses is by application of one layer of the material over the first passage zone and two or more layers of the same kind of material (i.e. in the same thickness). The layers may also be provided in different materials which may decompose or dissolve with different speeds - a fast dissolving and a slow dissolving. In another embodiment the first passage zone is made of a dissolvable material while the second passage zone is made of a decomposable material.

Behind the passage zone may be provided an indicator such as indicating colour and/or sign and/or picture which is visible when the passage zone is in the open state. Accordingly the opening of the passage zone may be used to indicate that waste material has reached a specific part to the ostomy appliance. As an example a part of the filter provided in the area of a passage zone may be coloured. In some embodiments the indicator is provided behind the passage zones which are used to open a passage from the collecting bag to the filter. Thus when the indicating colour is visible it is possible for flatus gasses to pass from the collecting bag through the passage zone, further through the filter and escape through an exit hole or membrane.

In another embodiment an indicating colour is provided close to the exit hole or membrane. From the outside of the appliance the indicating colour is not visible as it is covered by a decomposable or dissolvable passage zone. If waste material reaches that part of the filter which is close to the exit hole or membrane, the passage zone is decomposed or dissolved whereby the indicator is visible. Accordingly the user will be able to see that the appliance must be changed.

### Detailed description of the invention

An embodiment of the invention will now be described in details with reference to the drawings in which:
Figs. 1-3 show a passage zone and a filter according to the present invention,
Figs. 4a-c show a collecting bag with a first and second compartment interconnected by a passage zone, and
Figs. 5-7 show an ostomy appliance according to the present invention.

### Best mode of carrying out the invention

The invention is now explained more in detail with reference to the drawings showing preferred embodiments of the invention.

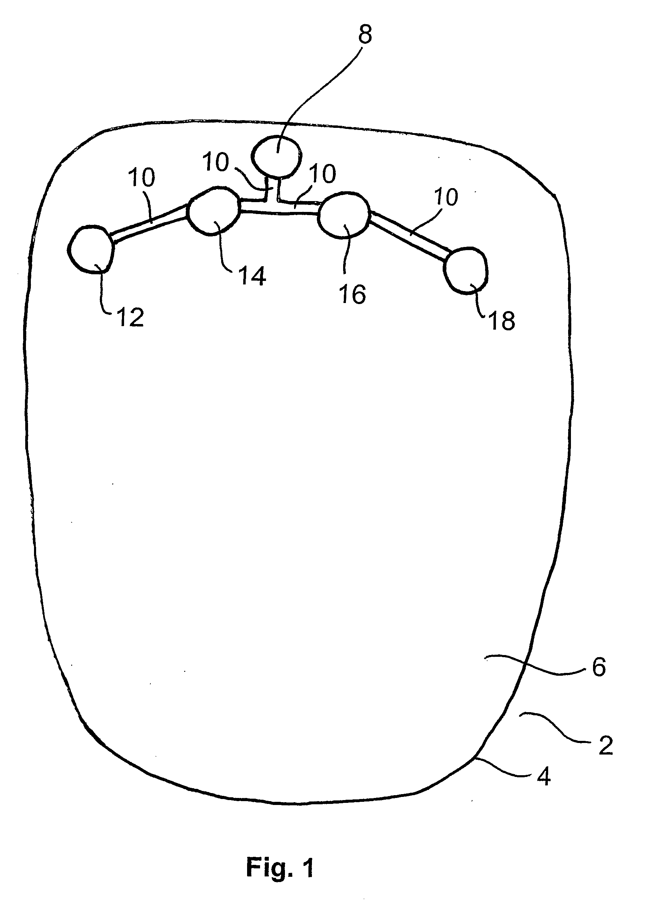

Fig. 1 discloses an ostomy appliance 2 according to the present invention. The ostomy appliance comprises a collecting bag 4 having a front wall 6 and a rear wall (not visible as the figure is a front view of the appliance). The ostomy appliance 2 comprises a filter 8 through which gasses may pass. The filter is interconnected with a passage system 10. One initial hole 12 is provided initially in an open state and a plurality of passage zones 14, 16, 18 are provided. When the initial hole 12 is blocked by waste material in solid or semi-solid form, gasses can not pass through said hole. After a predetermined period of time the first passage zone 14 opens as it dissolves or decompose when being in contact with the waste material. Afterwards the second passage zone 16 opens, and later the third passage zone 18 opens.

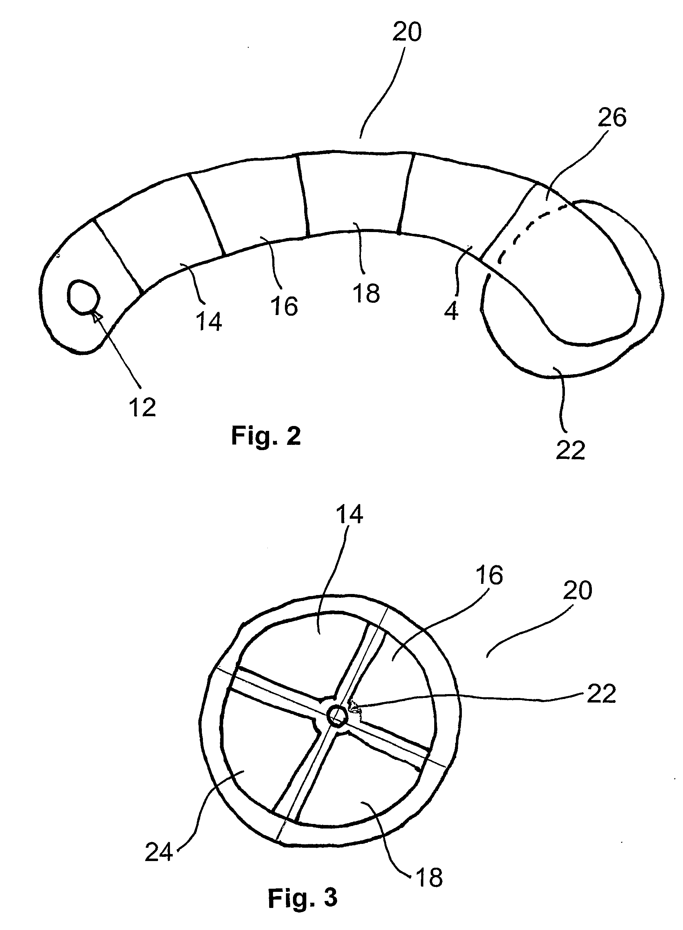

Fig. 2 shows a filter system 20 according to the present invention. The filter system 20 comprises an initial hole 12 and an exit hole 22 provided on the opposite of the filter system 20. A wall covering the filter system is divided into a plurality of passage zones 14, 16, 18, 24 and 26. The passage zones may open as described above and in the following sequence:
- first passage zone 14 after one hour,
- second passage zone 16 after eight hours,
- third passage zone 18 after 14 hours,
- fourth passage zone 24 after 20 hours, and
- fifth passage zone 26 after 30 hours.
The time period of each passage zone are examples and could be designed differently, e.g. such that they overlap. In an alternative embodiment the system is designed such that the passages zones open and the filter close to the passage zone is blocked in the following way.

| Passage zone | opens after | may be blocked after |
|---|---|---|
| First | 1 hour | 6 hours |
| Second | 3 hours | 12 hours |
| Third | 10 hours | 16 hours |
| Fourth | 13 hours | 24 hours |
| Fifth | 20 hours | 30 hours. |

Fig. 3 show an alternative of fig. 2 wherein the filter system 20 is circular and comprises a exit hole 22 and a plurality of passage zones 14, 16, 18 and 24. The passage zones are separated by attachment lines 28.

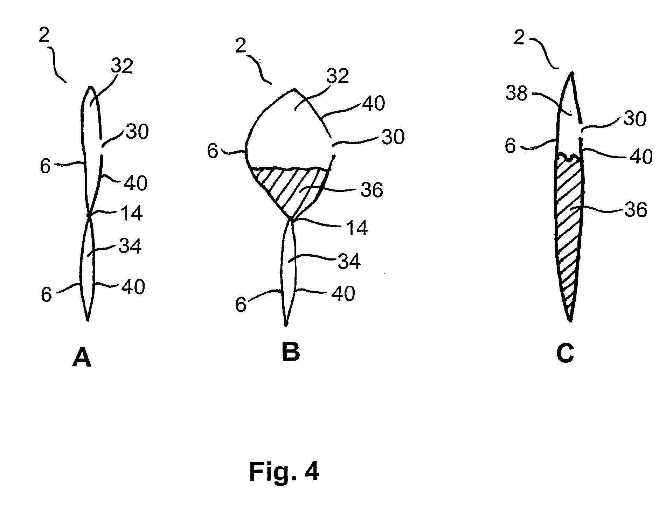

Fig 4a-c discloses an ostomy appliance 2 comprising an opening 30. The ostomy appliance 2 comprises a first compartment 32 and a second compartment 34 interconnected by a first passage zone 14. In figure 4a the appliance is empty and the first compartment 32 and the second compartment 34 constitute two separate compartments. In fig. 4b waste material 36 enter through the opening 30. After a period of time the first passage zone 14 dissolve or decompose and the first compartment 32 and the second compartment 34 turn into one joint compartment 38 thus making the appliance less thick as the distance from the front wall 6 to the rear wall 40 is shorter than in fig. 4b wherein the passage zone 14 is not open.

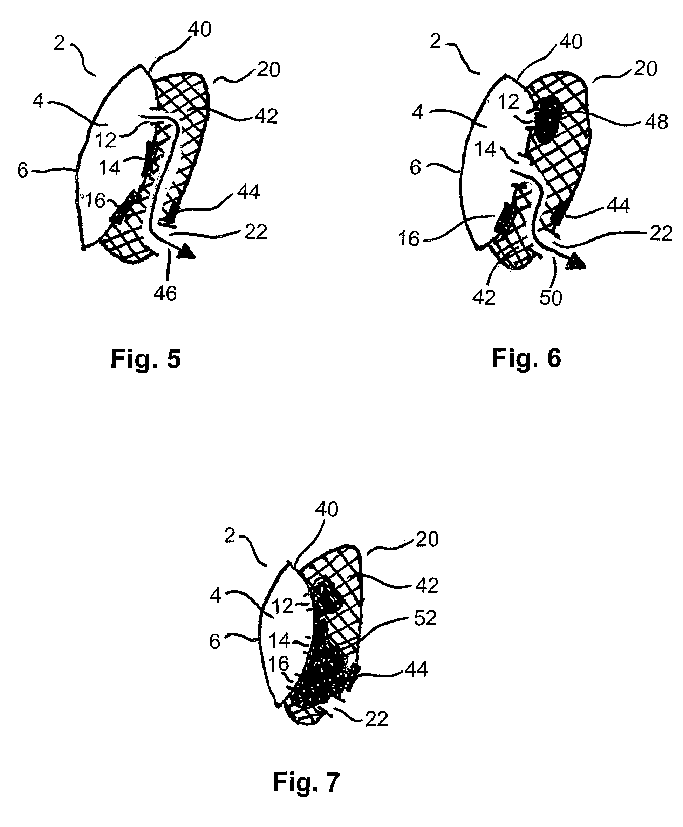

In figs. 5-7 a top view of the ostomy appliance 2 comprising a collecting bag 4 is provided. The ostomy appliance 2 comprises a front wall 6, a rear wall 40 and a filter system 20 comprising an initial hole 12 and an exit hole 22 provided on the opposite side of the filter 42. Furthermore first passage zone 14 and second passage zone 16 are provided. Finally the ostomy appliance comprises an indicating passage zone 44. Initially the initial hole 12 is open and gasses may pass as indicated by arrow 46 in fig. 5. After a period of time the initial hole 12 is blocked by waste material as indicated by blockage 48 in fig. 6. At this time first passage zone 14 is dissolved or decomposed and is now open, whereby gasses may follow the route indicated by arrow 50. After further time the first passage zone 14 is blocked and the second passage zone 16 opens. In the end a larger blockage 52 of the second passage zone 16 and the first passage zone will reach the indicating passage zone 44, which then opens such that an indicator is visible whereby the user is able to see that the appliance must be changed. As may be seen from the figures the first passage zone 14 and the second passage zone 16 are provided inside the appliance and thus the user may not open the passage zones e.g. by accident.

## Claims

1. An ostomy appliance (2) comprising a collecting bag (4) having a front wall (6) and a rear wall (40), the rear wall (40) having an opening (30) into the bag (4) through which waste material may enter the collecting bag (4), **characterized in that** at least one of the walls (6, 40) comprises at least one passage zone (14, 16, 18, 24, 26) adapted to change from a closed state to an open state, when exposed to the waste material.

2. An ostomy appliance (2) according to claim 1, wherein at least a part of the passage zone (14, 16, 18, 24, 26) is adapted to decompose and/or dissolve when exposed to the waste material.

3. An ostomy appliance (2) according to any of the preceding claims, wherein the surface of at least one passage zone (14, 16, 18, 24, 26) comprises a penetrable layer adapted to allow penetration of the liquid and a creasable layer adhered to the penetrable layer by means of a dissolvable adhesive.

4. An ostomy appliance (2) according to any of the preceding claims, wherein the collecting bag (4) comprises a first compartment (32) and a second compartment (34) separated by a passage zone (14, 16, 18, 24, 26).

5. An ostomy appliance (2) according to any of the preceding claims, further comprising a filter (8) through which gasses may pass.

6. An ostomy appliance (2) according to any of the preceding claims, wherein the passage zone (14, 16, 18, 24, 26) in the open state allows gasses to pass through the filter (8).

7. An ostomy appliance (2) according to any of the preceding claims, wherein a first passage zone (14) is adapted to change from the closed state to the open state after being exposed to the waste material for a predetermined first period of time.

8. An ostomy appliance (2) according to any of the preceding claims, wherein a second passage zone (16) is adapted to change from the closed state to the open state after being exposed to the waste material for a predetermined second period of time.

9. An ostomy appliance (2) according to any of the preceding claims, wherein the thickness of the wall of the first passage zone (14) in the closed state is smaller than the thickness of the wall of the second passage zone (16) in the closed state.

10. An ostomy appliance (2) according to any of the preceding claims, wherein an indicator (44) is visible when the passage zone (14, 16, 18, 24, 26) is in the open stage.

## Patentansprüche

1. Stomavorrichtung (2), die einen Sammelbeutel (4) aufweist, der eine vordere Wand (6) und eine hintere Wand (40) besitzt, wobei die hintere Wand (40) eine in den Beutel (4) führende Öffnung (30) aufweist, durch die Ausscheidungsmaterial in den Sammelbeutel (4) eintreten kann, **dadurch gekennzeichnet, dass** mindestens eine der Wände (6, 40) mindestens eine Durchgangszone (14, 16, 18, 24, 26) aufweist, die so ausgebildet ist, dass sie sich, wenn sie dem Ausscheidungsmaterial ausgesetzt ist, von einem geschlossenen Zustand in einen offenen Zustand ändert.

2. Stomavorrichtung (2) nach Anspruch 1, bei der zumindest ein Teil der Durchgangszone (14, 16, 18, 24, 26) so ausgebildet ist, dass er sich zersetzt und/oder auflöst, wenn er dem Ausscheidungsmaterial ausgesetzt ist.

3. Stomavorrichtung (2) nach einem der vorhergehenden Ansprüche, bei der die Oberfläche der mindestens einen Durchgangszone (14, 16, 18, 24, 26) eine penetrierbare Schicht, die so ausgebildet ist, dass sie die Penetration von Flüssigkeit erlaubt, und eine ablösbare Schicht aufweist, die durch einen auflösbaren Kleber auf der penetrierbaren Schicht aufgeklebt ist.

4. Stomavorrichtung (2) nach einem der vorhergehenden Ansprüche, bei welcher der Sammelbeutel (4) ein erstes Compartment (32) und ein zweites Compartment (34) aufweist, die durch eine Durchgangszone (14, 16, 18, 24, 26) getrennt sind.

5. Stomavorrichtung (2) nach einem der vorhergehenden Ansprüche, die ferner ein Filter (8) aufweist, durch das Gase hindurchtreten können.

6. Stomavorrichtung (2) nach einem der vorhergehenden Ansprüche, bei der die Durchgangszone (14, 16, 18, 24, 26) in offenem Zustand den Durchtritt von Gasen durch das Filter (8) erlaubt.

7. Stomavorrichtung (2) nach einem der vorhergehenden Ansprüche, bei der eine erste Durchgangszone (14) so ausgebildet ist, dass sie sich vom geschlossenen Zustand in den offenen Zustand ändert, nachdem sie dem Ausscheidungsmaterial für eine vorgegebene erste Zeitdauer ausgesetzt war.

8. Stomavorrichtung (2) nach einem der vorhergehenden Ansprüche, bei der eine zweite Durchgangszone (16) so ausgebildet ist, dass sie sich vom geschlossenen Zustand in den offenen Zustand ändert, nachdem sie dem Ausscheidungsmaterial für eine vorgegebene zweite Zeitdauer ausgesetzt war.

9. Stomavorrichtung (2) nach einem der vorhergehenden Ansprüche, bei der die Dicke der Wand der ersten Durchgangszone (14) in geschlossenem Zustand kleiner ist als die Dicke der Wand der zweiten Durchgangszone (16) in geschlossenem Zustand.

10. Stomavorrichtung (2) nach einem der vorhergehenden Ansprüche, bei der ein Indikator (44) sichtbar ist, wenn sich die Durchgangszone (14, 16, 18, 24, 26) in offenem Zustand befindet.

## Revendications

1. Dispositif pour stomie (2) comportant une poche collectrice (4), avec une paroi avant (6) et une paroi arrière (40), la paroi arrière (40) comportant une ouverture (30) dans la poche (4) au travers de laquelle les déchets peuvent pénétrer dans la poche collectrice (4), **caractérisé en ce qu'**au moins une des parois (6, 40) comprend au moins un passage (14, 16, 18, 24, 26), adapté à passer d'un état fermé à un état ouvert, quand il est exposé à des déchets.

2. Dispositif pour stomie (2) selon la revendication 1, dans lequel au moins une partie de la zone de passage (14, 16, 18, 24, 26) est adaptée à se décomposer et/ou se dissoudre quand elle est exposée aux déchets.

3. Dispositif pour stomie (2) selon l'une quelconque des revendications précédentes, dans lequel la surface d'au moins une zone de passage (14, 16, 18, 24, 26) comprend une couche pénétrable adaptée à permettre la pénétration du liquide et une couche froissable ayant adhéré à la couche pénétrable au moyen d'un adhésif soluble.

4. Dispositif pour stomie (2) selon l'une quelconque des revendications précédentes, dans lequel la poche collectrice (4) comporte un premier compartiment (32) et un deuxième compartiment (34) séparés par une zone de passage (14, 16, 18, 24, 26).

5. Dispositif pour stomie (2) selon l'une quelconque des revendications précédentes, comprenant en outre un filtre (8) à travers lequel peuvent circuler les gaz.

6. Dispositif pour stomie (2) selon l'une quelconque des revendications précédentes, dans lequel la zone de passage (14, 16, 18, 24, 26) à l'état ouvert permet aux gaz de passer à travers le filtre (8).

7. Dispositif pour stomie (2) selon l'une quelconque des revendications précédentes, dans lequel une première zone de passage (14) est adaptée à passer de l'état fermé à l'état ouvert après avoir été exposée aux déchets pendant une première durée prédéterminée.

8. Dispositif pour stomie (2) selon l'une quelconque des revendications précédentes, dans lequel une deuxième zone de passage (16) est adaptée à passer de l'état fermé à l'état ouvert après avoir été exposée aux déchets pendant une deuxième durée prédéterminée.

9. Dispositif pour stomie (2) selon l'une quelconque des revendications précédentes, dans lequel l'épaisseur de la paroi de la première zone de passage (14) à l'état fermé est inférieure à l'épaisseur de la paroi de la deuxième zone de passage (16) à l'état fermé.

10. Dispositif pour stomie (2) selon l'une quelconque des revendications précédentes, dans lequel un indicateur (44) est visible quand la zone de passage (14, 16, 18, 24, 26) est au stade ouvert.
